# EUROPEAN PATENT SPECIFICATION

(11) **EP 2 349 142 B1**
(45) Date of publication and mention of the grant of the patent: **07.01.2015**
(21) Application number: 09819878.1
(22) Date of filing: 08.10.2009
(51) Int. Cl.: A61F 6/04

(54) **LUBRICATED CONDOM**
KONDOM MIT GLEITMITTEL
PRÉSERVATIF LUBRIFIÉ

(30) Priority: 08.10.2008 US 103601 P; 14.11.2008 US 114503 P
(43) Date of publication of application: 03.08.2011
(73) Proprietor: Hui, Lap Shun, California 90804 (US)
(72) Inventor: Hui, Lap Shun, California 90804 (US)
(74) Representative: Beattie, Alex Thomas Stewart
(86) International application number: PCT/US2009/059991
(87) International publication number: WO 2010/042712

(56) References cited:
- JP-A- 7 289 576
- US-A1- 2006 081 264
- US-A1- 2006 134 611
- US-B1- 6 569 083

## Description

### FIELD OF THE INVENTION

The present invention is directed generally to a lubricated condom.

### BACKGROUND OF THE INVENTION

Sanitary contraceptives, such as condoms, are primarily designed to prevent the transfer of disease and unwanted pregnancy. Condoms have also been designed to provided stimulation through mechanical, chemical and visual means. Ribbed condoms that glow in the dark may provide both a mechanical an visual stimulation, for example. Further, use of external lubricants have been employed to increase satisfaction and ease of use of both the user of the condom and the user's target subject.

However, the lubrications employed historically fails to provide a sufficient amount of lubricant and the providing of the lubrication in a timely manner. If a lubricant is only provided at the initiation of use, the lubricating effects of the provided lubricant may diminish such that no practical lubrication is provided by the condom for a period of time before use ends. This may cause irritation for the users of the condom and may cause the condom to become dislodged.

Thus, there exists a need for a condom which can provide lubrication semi-continuously during use and not just a set amount prior to use.

### SUMMARY OF THE INVENTION

The present invention is directed to a condom, including a non-porous sheath member, having an open end and a rounded end, and shaped to cover the glans and body portion of a penis, and at least one rib having at least one outer portion and at least one inner portion, wherein the at least one outer portion is attachedly fixed to said non-porous sheath member, and wherein an inner cylinder formed by at the at least one inner portion is exclusive from said non-porous sheath member, and including at least one lubricant within the inner cylinder and at least one hole extending from the inner cylinder through the outer portion to provide for flow of the lubricant outwardly from the inner cylinder through the at least one hole pursuant to application of a predetermined pressure, wherein the at least one rib extends at least one of substantially from the open end to the closed end and substantially about a circumference of the non-porous sheath member.

The present invention solves problems experienced with the prior art because it provides lubrication semi-continuously during use and not just a set amount prior to use. Those and other advantages and benefits of the present invention will become apparent from the detailed description of the invention hereinbelow.

### BRIEF DESCRIPTION OF THE FIGURES

The present invention will be described hereinbelow in conjunction with the following figures, in which like numerals represent like items, and wherein:
Figure 1 illustrates an exemplary embodiment of the present invention;
Figure 2 illustrates a cross-section of at least one rib;
Figure 3 illustrates an exemplary embodiment of the present invention; and
Figure 4 illustrates a cross-section of the present invention.

### DETAILED DESCRIPTION OF THE INVENTION

It is to be understood that the figures and descriptions of the present invention have been simplified to illustrate elements that are relevant for a clear understanding of the present invention, while eliminating, for purposes of clarity, many other elements found in typical condoms. Those of ordinary skill in the art will recognize that other elements are desirable and/or required in order to implement the present invention. However, because such elements are well known in the art, and because they do not facilitate a better understanding of the present invention, a discussion of such elements is not provided herein.

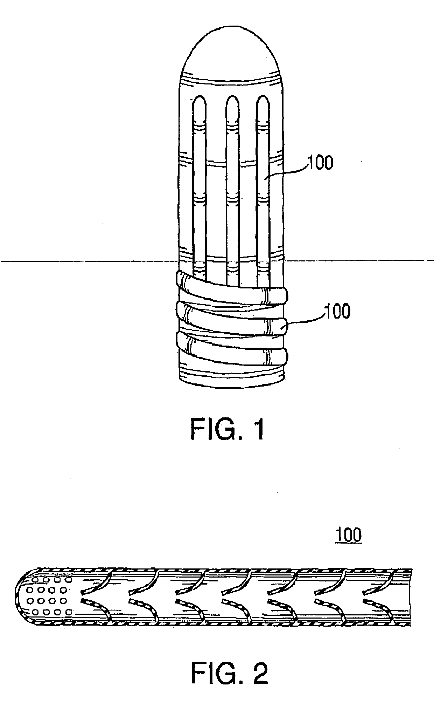

Figure 1 illustrates a condom in accordance with the present invention. As illustrated, the condom may include two or more ribs 100 along the barrel of the condom. Such ribs may be integral to, or separate from, the length of the barrel of the condom, but in a preferred embodiment, the underlying aspects of the condom is to be intact and free of holes irrespective of any holes, openings, veins, bladders, or the like integral with the aforementioned ribs of the present invention.

As used herein, the term "ribs" includes any vein, bladder, pathway, or the like, extending along any portion of the length of the barrel of a condom. As such, a rib, as used herein, provides a path for the flow of lubricant in at least a partially substantially parallel direction to the barrel of the condom, although, in certain embodiments, such flow along a rib may additionally occur along a portion substantially perpendicular to the length of the barrel of the condom.

As more particularly illustrated in Figure 2, a rib may allow for a flow of lubricant along or around the barrel of the condom. Such flow of lubricant may, in certain preferred embodiments, be directional, i.e., one way, at least in that the rib may include veins, valves, bladders, or the like that direct the flow of lubricant, such as upward from the base of the condom toward the tip of the condom during use of the condom. Such ribs may additionally include, such as along the length of the rib or at or near a termination of a rib, an exit pathway, such as a hole or holes, that allow lubricant to exit the condom. As discussed herein above, and in accordance with the present invention, it almost goes without saying that such holes or exit pathways should in no way interact with, pierce, or otherwise allow leakage from any portion of the underlying body of the condom, thereby preventing any leakage or penile discharge from inside the condom.

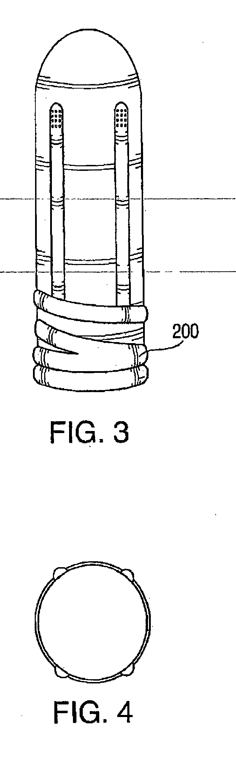

As further illustrated in Figure 3, the ribs of the present invention may provide a pathway at the base of the condom to one or more reservoirs 200 containing the aforementioned lubricant, which may, needless to say, be spermicidal lubricant. For example, pressure exerted along the barrel of the condom and focused at the base of the condom may force lubricant out of the one or more reservoirs and along the one directional ribs outward toward the upper tip of the condom. Such reservoirs may simply be one, two, three, or more spirals perpendicularly around the base of the condom, filled with lubricant, and sealed at the end of the reservoir spiral closest to the base of the condom. Thereby, when force is exerted at the base of the condom, the lubricant may, in accordance with a predetermined desired flow rate, flow around the spiral reservoir or reservoirs and upward along the barrel of the condom during use. Similarly, a single reservoir may be placed laterally around or approximate to the base of the condom, and such single reservoir may or may not be connected to the aforementioned spiral ribs, wherein, upon exertion of pressure downward along the barrel of the condom toward the base, pressure is exerted on the lubricant and the lubricant is forced from the reservoir and upward through the aforementioned ribs.

At present, the use of condoms presents various difficulties remedied by the present invention. For example, in prior art embodiments, lubrication provided on the condom may be providing in quantities greater than necessary in order to provide initial lubrication. Thereby, excess lubrication may reach the inside of the condom, such as when the condom is rolled up, and may thus cause the condom to be lose or difficult to use. This may particularly be the case when, in use, additional lubrication is added to the exterior of the condom. For example, such additional lubrication may simply work its way down to the base of the condom and have little effect during usage. As will be apparent to those skilled in the art in light of the discussion herein, the present invention remedies such issues. For example, the reserve lubrication provided in the present invention may be, in effect, time released, and may be released at the position most in need of lubrication during use of the condom, is, at the tip of the condom. As such, the present invention may be particularly useful in remedying the dryness that typically occurs after longer periods of usage of a condom. Likewise, the time release or targeted release of lubrication in the condom of the present invention may be prevent buildup of lubrication in undesired areas, that is, in those areas that may cause slippage of the condom, or difficulty in unrolling the condom.

Needless to say, the present invention may be provided with multiple reserves of lubricant, such as wherein each reservoir is separately time released, or is subject to release under different pressures, or subject to simultaneous release. Such multiple reservoirs may be used to create, for example, a ribbed effect for additional pleasure during use, as well as providing additional targeted lubricant during the use of the present invention.

The disclosure herein is directed to the variations and modifications of the elements of the invention disclosed that will be apparent to those skilled in the art in light of the disclosure herein. Thus, it is intended that the present invention covers the modifications and variations of this invention, provided those modifications and variations come within the scope of the appended claims.

## Claims

1. A condom, comprising:
a non-porous sheath member, having an open end and a rounded end, and shaped to cover the glans and body portion of a penis; **characterised by**
at least one rib (100) having at least one outer portion and at least one inner portion, wherein the at least one outer portion is attachedly fixed to said non-porous sheath member, and wherein an inner cylinder formed by the at least one inner portion is exclusive from said non-porous sheath member, and comprising at least one lubricant within the inner cylinder and at least one hole extending from the inner cylinder through the outer portion to provide for flow of the lubricant outwardly from the inner cylinder through the at least one hole pursuant to application of a pressure;
wherein the at least one rib (100) extends at least one of:
substantially from the open end to the closed end; and
substantially about a circumference of the non-porous sheath member.

2. The condom of claim 1, wherein said rib (100) is substantially filled with said lubricant.

3. The condom of claim 1, wherein said rib (100) further comprises at least one valve wherein said valve prevents the flow of lubricant towards said open end.

4. The condom of claim 3, wherein said valve is within said inner cylinder.

5. The condom of claim 1, wherein said pressure is applied externally to said rib (100).

6. The condom of claim 1, wherein said sheath member comprises a thermoplastic elastic material.

7. The condom of claim 1, wherein said sheath member comprises a thermoplastic polyurethane elastomer.

8. The condom of claim 1, wherein said rib comprises a thermoplastic elastic material.

9. The condom of claim 1, wherein said rib comprises a thermoplastic polyurethane elastomer.

10. The condom of claim 1, wherein said sheath member comprises a material selected from the group consisting of: polyurethanes; polyether block amides; styrene-rubber-styrene block copolymers; polyesters; olefinic homopolymers and copolymers; and copolymers, composites and blends thereof.

11. The condom of claim 1, wherein said rib (100) comprises a material selected from the group consisting of: polyurethanes; polyether block amides; styrene-rubber-styrene block copolymers; polyesters; olefinic homopolymers and copolymers; and copolymers, composites and blends thereof.

12. The condom of claim 1, wherein said at lest one rib (100) is proximate to a roll at said open end of said sheath member.

13. The condom of claim 1, wherein said sheath member has a varying diameter penis receiving compartment.

14. The condom of claim 1, further comprising a sanitary pouch encompassing said sheath member until use.

15. The condom of claim 1, wherein said sheath member is watertight, and wherein said rib (100) is not watertight.

## Patentansprüche

1. Kondom, umfassend:
ein nicht-poröses Hüllenelement, das ein offenes Ende und ein gerundetes Ende aufweist und so geformt ist, dass es die Eichel und den Körper eines Penis abdeckt;
**gekennzeichnet durch**
zumindest eine Rippe (100) mit zumindest einem Außenteil und zumindest einem Innenteil, worin das zumindest eine Außenteil fest an besagtem nicht-porösem Hüllenelement angebracht ist, und worin ein von dem zumindest einem Innenteil ausgebildeter innerer Zylinder exklusiv aus besagtem nicht-porösem Hüllenelement besteht, und umfassend zumindest ein Gleitmittel innerhalb des inneren Zylinders und zumindest ein Loch, das sich von dem inneren Zylinder **durch** das Außenteil erstreckt, um für das Fließen des Gleitmittels nach außen von dem inneren Zylinder durch das zumindest eine Loch entsprechend der Ausübung eines Drucks zu sorgen;
worin sich die zumindest eine Rippe (100) zumindest entweder:
im Wesentlichen von dem offenen Ende zu dem geschlossenen Ende erstreckt; oder
im Wesentlichen um einen Umfang des nicht-porösen Hüllenelements herum erstreckt.

2. Kondom nach Anspruch 1, worin besagte Rippe (100) im Wesentlichen mit besagtem Gleitmittel gefüllt ist.

3. Kondom nach Anspruch 1, worin besagte Rippe (100) ferner zumindest ein Ventil umfasst, worin besagtes Ventil das Fließen von Gleitmittel hin zu besagtem offenem Ende verhindert.

4. Kondom nach Anspruch 3, worin besagtes Ventil innerhalb des besagten inneren Zylinders ist.

5. Kondom nach Anspruch 1, worin besagter Druck extern auf besagte Rippe (100) ausgeübt wird.

6. Kondom nach Anspruch 1, worin besagtes Hüllenelement aus einem thermoplastischen elastischen Material besteht.

7. Kondom nach Anspruch 1, worin besagtes Hüllenelement aus einem thermoplastischen Polyurethan-Elastomer besteht.

8. Kondom nach Anspruch 1, worin besagte Rippe aus einem thermoplastischen elastischen Material besteht.

9. Kondom nach Anspruch 1, worin besagte Rippe aus einem thermoplastischen Polyurethan-Elastomer besteht.

10. Kondom nach Anspruch 1, worin besagtes Hüllenelement aus einem Material besteht, das aus der aus Folgendem bestehenden Gruppe ausgewählt wird: Polyurethanen; Polyetherblockamiden; Styrol-Gummi-Styrol-Blockcopolymeren; Polyestern; olefinischen Homopolymeren und Copolymeren; und Copolymeren, Verbundstoffen und Gemischen davon.

11. Kondom nach Anspruch 1, worin besagte Rippe (100) aus einem Material besteht, das aus der aus Folgendem bestehenden Gruppe ausgewählt wird: Polyurethanen; Polyetherblockamiden; Styrol-Gummi-Styrol-Blockcopolymeren; Polyestern; olefinischen Homopolymeren und Copolymeren; und Copolymeren, Verbundstoffen und Gemischen davon.

12. Kondom nach Anspruch 1, worin besagte zumindest eine Rippe (100) nahe bei einer Rolle an besagtem offenem Ende von besagtem Hüllenelement ist.

13. Kondom nach Anspruch 1, worin besagtes Hüllenelement ein den Penis aufnehmendes Kompartiment mit veränderlichem Durchmesser aufweist.

14. Kondom nach Anspruch 1, ferner umfassend einen sanitären Beutel, der besagtes Hüllenelement bis zum Gebrauch umschließt.

15. Kondom nach Anspruch 1, worin besagtes Hüllenelement wasserdicht ist und worin besagte Rippe (100) nicht wasserdicht ist.

## Revendications

1. Préservatif, comprenant : un élément de gaine non poreux, comportant une extrémité ouverte et une extrémité arrondie, et dont la forme recouvre le gland et une partie de corps d'un pénis ; **caractérisé par**
au moins une nervure (100) comportant au moins une partie extérieure et au moins une partie intérieure, ladite partie extérieure étant fixée de manière non amovible audit élément de gaine non poreux, et un cylindre intérieur formé par ladite partie intérieure est distinct dudit élément de gaine non poreux, et comprenant au moins un lubrifiant dans le cylindre intérieur et au moins un orifice s'étendant du cylindre intérieur à la partie extérieure afin de permettre l'écoulement du lubrifiant vers l'extérieur à partir du cylindre intérieur à travers ledit orifice suite à l'application d'une pression ;
ladite nervure (100) s'étendant au moins :
pratiquement de l'extrémité ouverte à l'extrémité fermée ; et
pratiquement par rapport à une circonférence de l'élément de gaine non poreux.

2. Préservatif selon la revendication 1, dans lequel ladite nervure (100) est pratiquement remplie avec ledit lubrifiant.

3. Préservatif selon la revendication 1, dans lequel ladite nervure (100) comprend en outre au moins un clapet, ledit clapet empêchant l'écoulement de lubrifiant vers ladite extrémité ouverte.

4. Préservatif selon la revendication 3, dans lequel ledit clapet se trouve à l'intérieur dudit cylindre intérieur.

5. Préservatif selon la revendication 1, dans lequel ladite pression est appliquée extérieurement à ladite nervure (100).

6. Préservatif selon la revendication 1, dans lequel ledit élément de gaine est constitué d'un matériau élastique thermoplastique.

7. Préservatif selon la revendication 1, dans lequel ledit élément de gaine est constitué d'un élastomère de polyuréthane thermoplastique.

8. Préservatif selon la revendication 1, dans lequel ladite nervure est constituée d'un matériau élastique thermoplastique.

9. Préservatif selon la revendication 1, dans lequel ladite nervure est constituée d'un élastomère de polyuréthane thermoplastique.

10. Préservatif selon la revendication 1, dans lequel ledit élément de gaine est constitué d'un matériau choisi dans le groupe constitué de : polyuréthanes, amides blocs de polyéther, copolymères blocs de styrène-caoutchouc-styrène, polyesters, homopolymères et copolymères oléfiniques, et copolymères, composites et mélanges correspondants.

11. Préservatif selon la revendication 1, dans lequel ladite nervure (100) est constituée d'un matériau choisi dans le groupe constitué de : polyuréthanes, amides blocs de polyéther, copolymères blocs de styrène-caoutchouc-styrène, polyesters, homopolymères et copolymères oléfiniques, et copolymères, composites et mélanges correspondants.

12. Préservatif selon la revendication 1, dans lequel ladite nervure (100) est proche d'un rouleau situé à l'extrémité ouverte dudit élément de gaine.

13. Préservatif selon la revendication 1, dans lequel ledit élément de gaine comporte un compartiment de réception de pénis à diamètre variable.

14. Préservatif selon la revendication 1, comprenant en outre un étui sanitaire enfermant ledit élément de gaine jusqu'à son utilisation.

15. Préservatif selon la revendication 1, dans lequel ledit élément de gaine est étanche à l'eau, et dans lequel ladite nervure (100) n'est pas étanche à l'eau.
